# EUROPÄISCHE PATENTSCHRIFT

(11) **EP 0 750 502 B1**
(45) Veröffentlichungstag und Bekanntmachung des Hinweises auf die Patenterteilung: **02.05.2001**
(21) Anmeldenummer: 95911149.3
(22) Anmeldetag: 20.03.1995
(51) Int. Cl.: A61K 31/675

(54) **VERWENDUNG VON PHOSPHORDERIVATEN VON ALKALOIDEN ZUR BEHANDLUNG VON ENDOKRINOPATHIEN**
USE OF PHOSPHORUS DERIVATIVES OF ALKALOIDS FOR TREATING ENDOCRINOPATHIES
UTILISATION DE DERIVES PHOSPHORIQUES D'ALCALO DES POUR LE TRAITEMENT D'ENDOCRINOPATHIES

(30) Priorität: 18.03.1994 AT 57894
(43) Veröffentlichungstag der Anmeldung: 02.01.1997
(73) Patentinhaber: Nowicky, Wassyl, A-1040 Wien (AT)
(72) Erfinder: Nowicky, Wassyl, A-1040 Wien (AT)
(74) Vertreter: Pawloy, Heinrich, Dr.
(86) Internationale Anmeldenummer: AT9500055
(87) Internationale Veröffentlichungsnummer: WO9525522

(56) Entgegenhaltungen:
- EP-A- 0 326 627
- US-A- 4 816 462
- DRUGS UNDER EXPERIMENTAL AND CLINICAL RESEARCH, vol. 18, 1992 pages 93-96, KLEINROK, Z. ET AL 'SOME PHARMACOLOGICAL PROPERTIES OF PROLONGED ADMINISTRATION OF UKRAIN IN RODENTS'
- DRUGS UNDER EXPERIMENTAL AND CLINICAL RESEARCH, vol. 18, 1992 pages 85-87, JAGIELLO-WOJTOWICZ, E. ET AL 'EFFECT OF SINGLE AND THREE MONTHS TREATMENT WITH UKRAIN ON AMINOTRANSFERASES (ALT AND AST) AND ON THE SERUM PROTEIN LEVEL IN RODENTS'
- DRUGS UNDER EXPERIMENTAL AND CLINICAL RESEARCH, vol. 18, 1992 pages 89-91, JAGIELLO-WOJTOWICZ, E. ET AL 'EFFECT OF PROLONGED ADMINISTRATION OF UKRAIN ON PROLACTIN CONCENTRATION IN RATS'

## Beschreibung

Die vorliegende Erfindung betrifft die Verwendung von Phosphorderivaten von Alkaloiden zur Herstellung eines Arzneimittels zur Behandlung von Endokrinopathien.

Unter der Bezeichnung Endokrinopathien werden Krankheitsbilder verstanden, bei denen hormonelle Störungen ursätzlich und krankheitsbestimmend im Vordergrund stehen. Die Ursachen derartiger Krankheitsbilder können in Erkrankung der endokrinen Drüsen, beispielsweise vermehrte Hormonproduktion oder Hormonmangel oder Ausfall, in Funktionsstörungen der endokrinen Drüsen aufgrund regulatorischer Vorgänge, in entgleister Hormonbildung in Folge pathologischer Enzymsysteme oder in veränderter Ansprechbarkeit verschiedener Organe auf Hormone liegen.

Zu den Endokrinopathien kann unter anderem auch Osteoporose gezählt werden, wobei es sich dabei um die quantitative Verminderung des Knochengewebes bei erhaltener Knochenstruktur durch gesteigerten Knochenabbau und/oder verminderten -anbau, begleitet von vermehrtem Auftreten heparinhaltiger Mastzellen im Knochenmark handelt. Die Äthiologie dieser Krankheit ist zwar weitgehend ungeklärt, es bestehen jedoch starke Hinweise darauf, daß sie durch Östrogenmangel aufgrund des Eintretens der Menopause zumindest stark begünstigt wird.

In einem Artikel von H. Resch et al. (Calcif Tissue Int. (1989) 45:209-213) wird zur Behandlung von Osteoporose eine kombinierte Gabe von Calcitonin und zyklischer Hormonersatztherapie vorgeschlagen. Weiters wurde vom selben Autor in Acta Endocrinologica 1990, 123, S.14-18 zyklische Östrogen/Gestagenersatztherapien zur Behandlung von Osteoporose beschrieben. Die Ergebnisse dieser Studien zeigen, daß eine Hormonbehandlung bei Osteoporosepatienten erfolgversprechend scheint.

Auch B.E.C. Nordin beschreibt in Osteoporose, Verlag Wilhelm Maudrich, Wien-München-Bern, 1989, daß zumindest bei Frauen die mit Osteoporose einhergehende erhöhte Knochenresorption vermutlich auf einer abnehmenden Ovarialfunktion zurückzuführen ist, wobei nach der Menopause das von der Nebennierenrinde produzierte Androstendion die einzige Östrogenquelle ist, aus der wiederum eine geringe Menge an Östradiol produziert wird. Eine kleine Menge Östradiol entsteht durch periphere Umwandlung aus Testosteron, das wiederum teilweise aus dem Androstendion aus der Nebennierenrinde und teilweise im postmenopausalen Ovar gebildet wird. Angesichts dieses ziemlich komplexen Mechanismus ist es schwer zu sagen, welche hormonellen Veränderungen für die steigende Knochenresorption in der Menopause verantwortlich sind. Da diese durch eine Östrogentherapie reversibel ist, beruht sie wahrscheinlich auf der Abnahme der gesamten wirksamen Östrogenaktivität (Östradiol und Östron). Die Ovarialinsuffizienz muß aber nicht unbedingt eine direkte Auswirkung auf den Knochen haben; indirekt würde sie durch Veränderungen der Kalzitoninsekretion wirken. In der direkten postmenopausalen Phase steigt das Serum- und Harnkalzium sicherlich ohne Erhöhung der Kalziumresorption an, der Kalziumbedarf kann dabei sogar bis auf 35 mmol/Tag steigen. Zur Auswirkung verschiedener Hormone bzw. deren Mangel auf Osteoporose sei auf das letztgenannte Werk von B.E.C. Nordin verwiesen.

Die AT-PS-377 988 und die AT-PS-354 644 beschreiben Verfahren zur Herstellung von neuen Phosphorderivaten von Alkaloiden bzw. von neuen Salzen von Alkaloidderivaten von Thiophosphorsäure. Derartige Verbindungen besitzen eine pharmakologische Wirksamkeit und können als Cytostatika Verwendung finden.

Es wurde nun überraschend gefunden, daß die in der AT-PS 377 988 bzw. der AT-PS-354 644 geoffenbarten Phosphorderivate von Alkaloiden zur Herstellung von Arzneimittel zur Behandlung von Endokrinopathien, insbesondere zur Behandlung von Osteoporose verwendet werden können.

Verfahren zur Herstellung von Phosporderivaten von Alkaloiden der allgemeinen Formel (I) 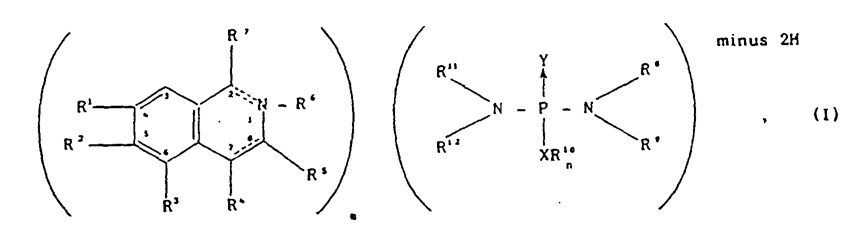 worin m und n = 1, 2 oder 3 ist; R¹, R² und R³ unabhängig voneinander jeweils H oder Methoxy bedeuten, wobei R¹ und R² oder R² und R³ zusammen auch eine Methylendioxygruppe darstellen können;
R⁴ und R⁵ zusammen mit den C-Atomen, an denen sie haften, eine gegebenenfalls ganz oder teilweise aushydrierte Phenyl- oder Naphthylgruppe bilden, die ihrerseits durch Methoxy, Hydroxy oder Dioxymethyl substituiert sein kann, wobei R⁷ H oder =O oder ein über eine -CH₂-CO-CH₂-Kette gebundenes gleiches Ringsystem ist, R⁶ -CH₃ bedeutet und Doppelbindungen in Stellung 1, 2 und/oder 7, 8 vorhanden sein können; oder
R⁶ und R⁷ zusammen mit dem C- und N-Atom, an denen sie haften, ein gegebenenfalls hydriertes Benzo- oder Naphthoringsystem bilden, das seinerseits durch Methoxy, Oxo, Methyl oder Dioxymethylgruppen substituiert sein kann, wobei die C-N-Bindung in Stellung 1, 2 fehlen kann und R⁴ und R⁵ H bedeuten;
R¹⁰ = 2H, -CH₂-CH₂-, H oder -CH₂-CH₂Cl bedeutet;
X = O oder W ist,
R⁸ + R⁹ und R¹¹ + R¹² -CH₂-CH₂- bedeuten und, wenn Y = S, X = N und n = 2, R² und R³ -CH₂-CH₂-, -CH₂-CH₂-O-CH₂-CH₂-, oder 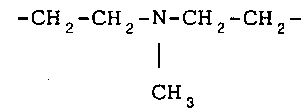 bedeutet; wenn
   Y = S, X = N, n = 2, R² und R³ -CH₂-CH₂-, 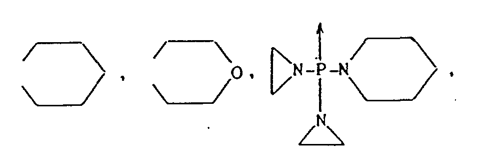 oder (-C₂H₅)₂, 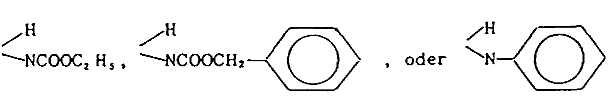 darstellt; wenn
   Y = S, X = O, n = 1, R³ 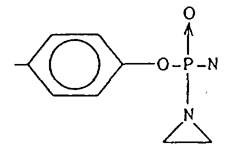 ist; wenn
   Y = O, X = N, n = 1, R³ 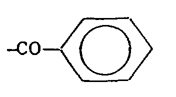 bedeutet;
   Y = O, X = N, n = 2, R² und R³ 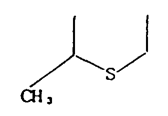 ist; und, wenn
   Y = O, X = O, n = 1,
R⁸ und R⁹ je -CH₂-CH₂-Cl, R¹⁰ H₂ und R¹¹ + R¹² -CH₂-CH₂- oder -CH₂-CH₂-CH₂- bedeuten, wenn Y = S, X = N, R³ -CH₂-CH₂- ist, sowie deren Salzen mit pharmazeutisch verträglichen Säuren, sind aus der AT-PS 377 988 bekannt; die Herstellung von Alkaloidderivaten von Thiophosphorsäure der allgemeinen Formel 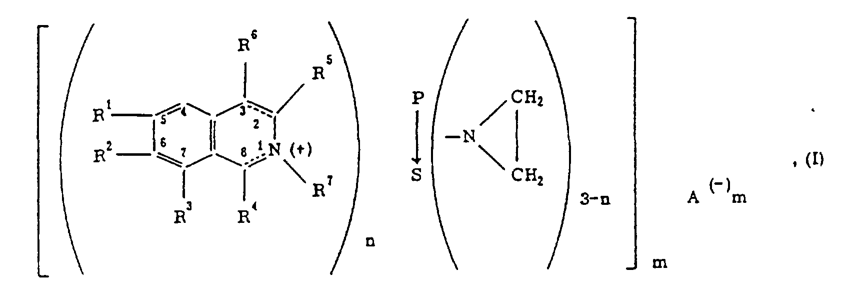 worin n 1, 2 oder 3 ist; m 1, 2 oder 3 bedeutet; R¹, R² und R³ unabhängig voneinander jeweils Wasserstoff oder Methoxy bedeuten, wobei R¹ und R² oder R² und R³ zusammen auch eine Methylendioxygruppe darstellen können; R⁴ Wasserstoff, Hydroxy oder Methyl bedeutet; und, wenn R⁶ Wasserstoff ist, R⁵ und R⁷ zusammen die Gruppe 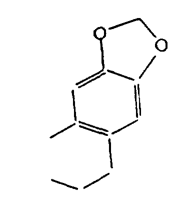 bilden: oder, wenn R⁷ eine Methylgruppe bedeutet die Gruppen R⁵ und R⁶ die Gruppe 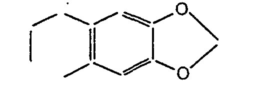 darstellen; und in Stellung 1, 8 und/oder 2, 3 eine Doppelbindung vorhanden sein kann; und A ein einwertiges oder den äquivalenten Teil eines mehrwertigen Anions bedeutet, wird in der AT-PS 354 644 geoffenbart.

Gemäß einer besonders bevorzugten Ausführungsform der vorliegenden Erfindung wird das Umsetzungsprodukt der Alkaloide von Chelidonium maijus L. mit Thiophosphorsäuretriaziridid zur Herstellung eines Arzneimittels zur Behandlung von Endokrinopathien, insbesondere zur Behandlung von Osteoporose, eingesetzt. Dieses Umsetzungsprodukt wird der Einfachheit halber nachfolgend mit "Umsetzungsprodukt" bezeichnet.

Die überraschende Wirkung dieses Umsetzungsproduktes bei der Behandlung von Endokrinopathien wird nachfolgend anhand eines Tierversuchsmodells belegt.

Dabei wurde die Langzeitwirkung des Umsetzungsproduktes auf einige biochemische und biomechanische Parameter in ovariektomierten Ratten untersucht.

Ovariektomie ist ein anerkanntes Modell für die experimentelle Osteoporose. Die Versuchstiere erhielten dabei intraperitoneale Injektionen vom Umsetzungsprodukt bei einer Dosis von 28 mg/kg Körpergewicht jeden Tag während 6 Monate ab dem zweiten Tag nach der Entfernung der Eierstöcke bzw. nach einer entsprechenden Operation ohne Entfernung der Eierstöcke (Kontrollgruppe mit Operationsschock). Die Ovariektomie verursachte Veränderungen in der peripheralen Blutmorphologie, der Aktivität von Aminotransferasen (ALT und AST) und im Gesamtserumproteinspiegel sowie in den Serum-hormonkonzentrationen und der Menge von Catecholaminen im gesamten Hirn der Ratten. Die Veränderungen werden im einzelnen in den nachfolgenden Tabellen angegeben.

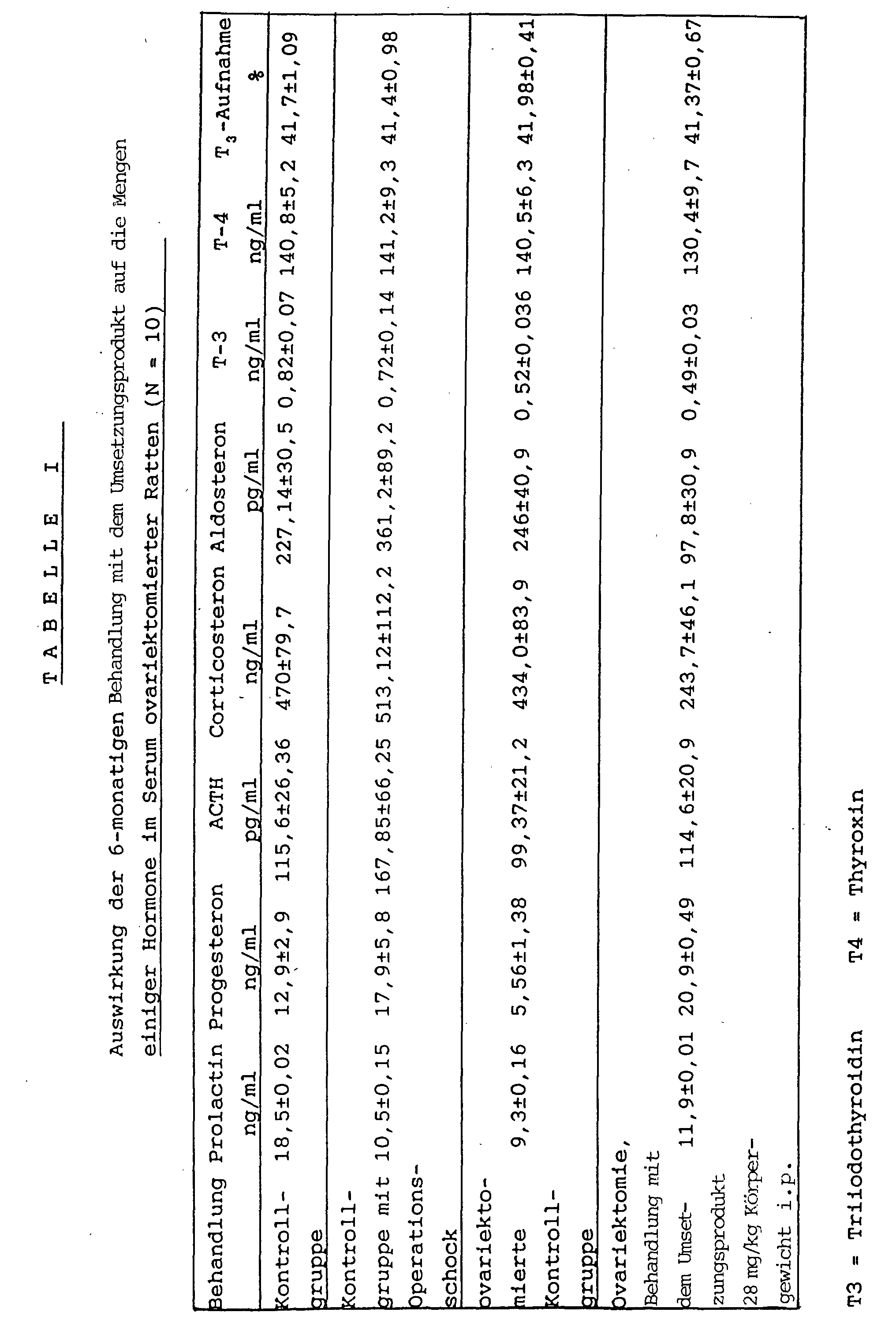

**TABELLE II**

| Auswirkung der 6-monatigen Behandlung mit dem Umsetzungsprodukt auf die Mengen von Noradrenalin (NA) und Dopamin (DA) im ganzen Hirn in ovariektomierten Ratten (N = 10) | | |
|---|---|---|
| Behandlung | ng/g Frischgewebe | |
| | NA | DA |
| Kontrollgruppe | 1,214 ± 0,043 | 0,778 ± 0,032 |
| Kontrollgruppe mit Operationsschock | 1,4703 ± 0,077 | 0,913 ± 0,061 |
| ovariektomierte Kontrollgruppe | 1,625 ± 0,064 | 1,0208 ± 0,047 |
| Ovariektomie, Behandlung mit dem Umsetzungsprodukt 28 mg/kg Körpergewicht i.p. | 1,274 ± 0,085 | 0,920 ± 0,027 |

**TABELLE III**

| Auswirkung der 6-monatigen Behandlung mit dem Umsetzungsprodukt auf die Aktivität von Aminotransferasen (ALT und AST) im Serum von ovariektomierten Ratten (N = 10) | | |
|---|---|---|
| Behandlung | Aktivität i.p. | |
| | ALT | AST |
| Kontrollgruppe | 25,3 ± 0,4 | 19 ± 0,6 |
| Kontrollgruppe mit Operationsschock | 25,8 ± 0,4 | 23,5 ± 0,5 |
| ovariektomierte Kontrollgruppe | 25,1 ± 0,6 | 21,3 ± 0,3 |
| Ovariektomie, Behandlung mit dem Umsetzungsprodukt 28 mg/kg Körpergewicht i.p. | 24 ± 0,4 | 19,4 ± 0,5 |

**TABELLE IV**

| Auswirkung von 6-monatiger Behandlung mit dem Umsetzungsprodukt auf die peripherale Blutmorphologie in ovariektomierten Ratten (N = 10) | | | | |
|---|---|---|---|---|
| Behandlung | Haemoglobin | Erythrocyten | Haematocrit | Leukocyten |
| | g % | 10⁶/mm³ | % | 10³/mm³ |
| Kontrollgruppe | 14,4 ± 0,14 | 7,5 ± 0,08 | 40,8 ± 0,3 | 14,4 ± 0,14 |
| Kontrollgruppe mit Operationsschock | 15,5 ± 0,2 | 8,18 ± 0,06 | 45,6 ± 0,17 | 15,5 ± 0,2 |
| ovariektomierte Kontrollgruppe | 15,5 ± 0,3 | 8,05 ± 0,02 | 44,9 ± 0,04 | 15,5 ± 0,3 |
| Ovariektomie, Behandlung mit dem Umsetzungsprodukt 28 mg/kg Körpergewicht i.p. | 14,8 ± 0,07 | 7,9 ± 0,03 | 43,2 ± 0,2 | 14,8 ± 0,07 |

Aus den obigen Tabellen I bis IV ist ersichtlich, daß die Veränderungen, welche das Umsetzungsprodukt bei ovariektomierten Ratten verursacht, dahingehend signifikant sind, daß alle Parameter, welche nach einer Ovariektomie und daher mit größter Wahrscheinlichkeit auch bei Osteoporose außerhalb der Norm liegen, verbessert werden.

In diesem Zusammenhang ist auch besonders bemerkenswert, daß sich nach der Behandlung und Sakrifizierung der Tiere eine signifikant bessere mechanische Bruchbelastbarkeit der Faemurknochen bei mit dem Umsetzungsprodukt behandelten Tieren im Vergleich zur ovariektomierten Kontrollgruppe ergab.

Als Alkaloidkomponente haben sich als besonders geeignet erwiesen: Coptisin, Stylopin, Berberin, Protopin, Allo-Kryptopin, Spartein, Corysamin, Chelidimerin, Oxysanguinarin, Sanguinarin, Dihydroxysanguinarin, Chelidonin, Homochelidonin, Methoxy-chelidonin, Chelerythrin, Chelilutin, Winblastin, Colchicin, Cholchicein, Desacetyl-N-methyl-colchinin.

Als Phosphorverbindung für die Umsetzung kommen insbesondere in Frage:

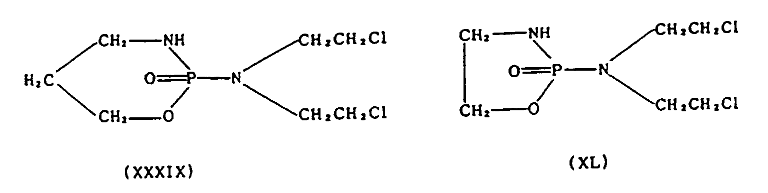

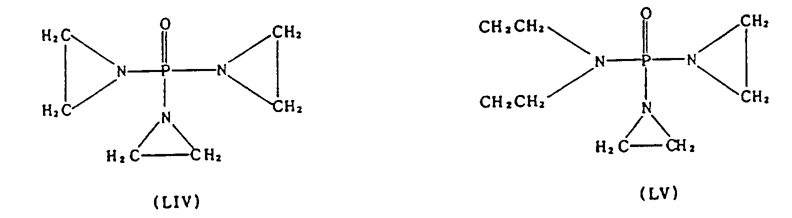

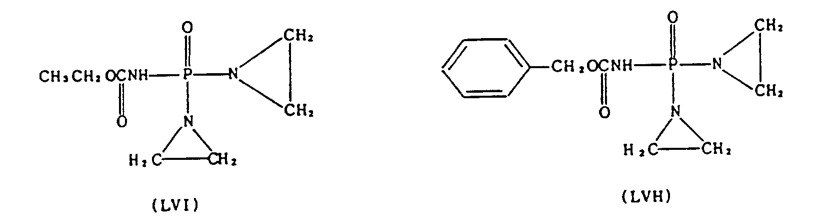

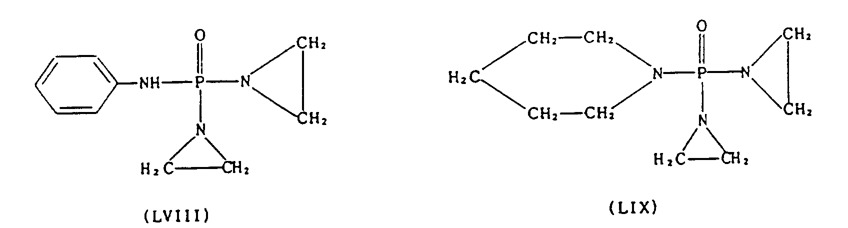

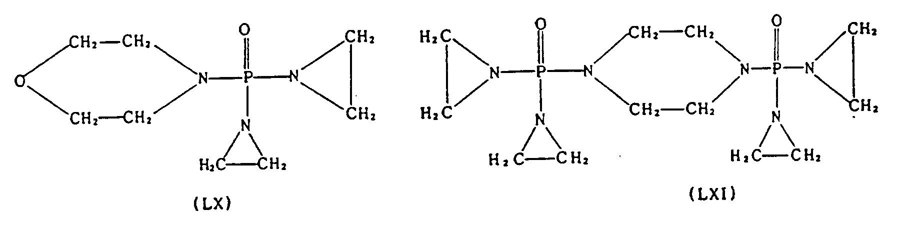

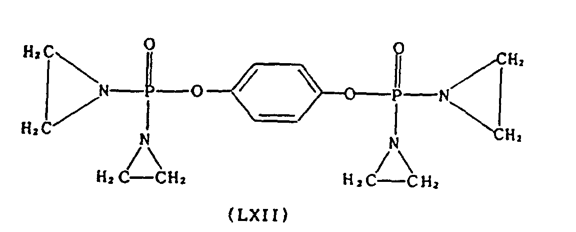

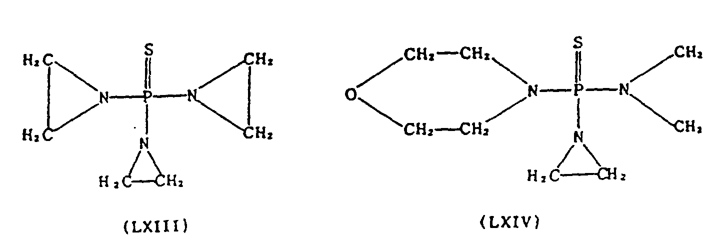

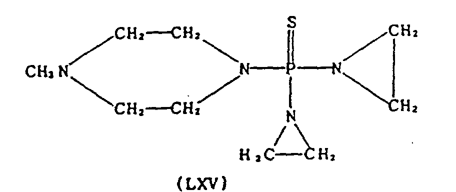

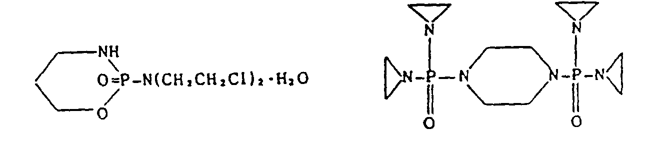

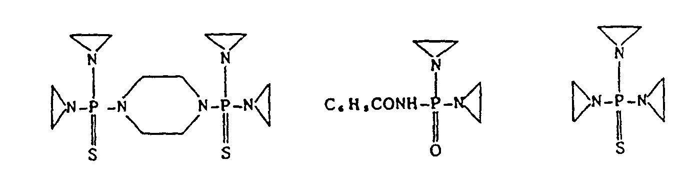

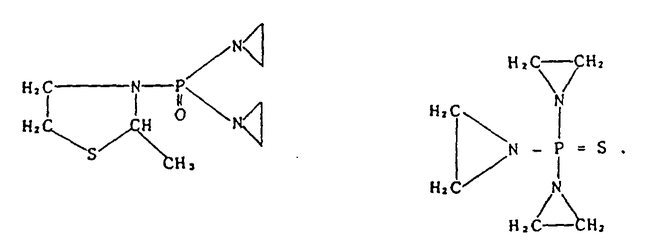

Die erfindungsgemäß hergestellten Arzneimittel bestehen vorzugsweise aus einer wässerigen Lösung der verwendeten Alkaloidphosphorderivate bzw. deren Salze, gegebenenfalls zusammen mit weiteren, an sich bekannten Hilfsmitteln. Die Verabreichung des erfindungsgemäßen Arzneimittels erfolgt dabei bevorzugt durch Injektion, beispielsweise intraperitoneal, intramuskulär oder intravenös, wobei die Dosierung fallabhängig ist und mit der Schwere der zu behandelnden Erkrankung sowie dem Zustand des Patienten zusammenhängt.

Es liegt jedenfalls im Fachwissen des behandelnden Arztes, eine geeignete Dosierung von Fall zu Fall zu bestimmen.

## Patentansprüche

1. Verwendung von Phosphorderivaten von Alkaloiden der allgemeinen Formel (I) 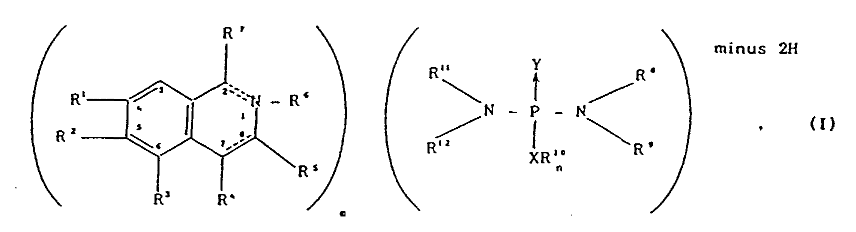 worin m und n = 1, 2 oder 3 ist; R¹, R² und R³ unabhängig von-ein-ander jeweils H oder Methoxy bedeuten, wobei R¹ und R² oder R² und R³ zusammen auch eine Methylendioxygruppe darstellen können;
R⁴ und R⁵ zusammen mit den C-Atomen, an denen sie haften, eine gegebenenfalls ganz oder teilweise aushydrierte Phenyl- oder Naphthylgruppe bilden, die ihrerseits durch Methoxy, Hydroxy oder Dioxymethyl substituiert sein kann, wobei R⁷ H oder =O oder ein über eine -CH₂-CO-CH₂-Kette gebundenes gleiches Ringsystem ist, R⁶ -CH₃ bedeutet und Doppelbindungen in Stellung 1, 2 und/oder 7, 8 vorhanden sein können; oder
R⁶ und R⁷ zusammen mit dem C- und N-Atom, an denen sie haften, ein gegebenenfalls hydriertes Benzo- oder Naphthoringsystem bilden, das seinerseits durch Methoxy, Oxo, Methyl oder Dioxymethylgruppen substituiert sein kann, wobei die C-N-Bindung in Stellung 1, 2 fehlen kann und R⁴ und R⁵ H bedeuten;
R¹⁰ = 2H, -CH₂-CH₂-, H oder -CH₂-CH₂Cl bedeutet;
X = O oder N ist,
R⁸ + R⁹ und R¹¹ + R¹² -CH₂-CH₂- bedeuten und, wenn Y = S, X = N und n = 2, R² und R³ -CH₂-CH₂-, -CH₂-CH₂-O-CH₂-CH₂-, oder 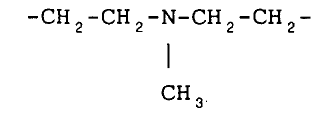 bedeutet; wenn
Y = S, X = N, n = 2, R² und R³ -CH₂-CH₂-, 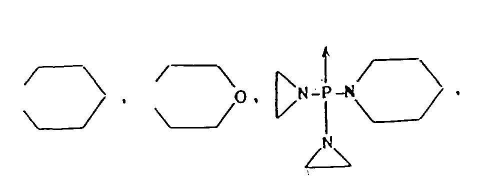 oder (-C₂H₅)₂, 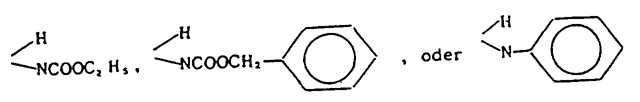 darstellt; wenn
Y = S, X = O, n = 1, R³ 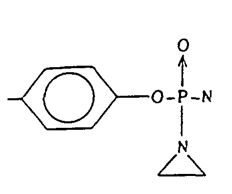 ist; wenn
Y = O, X = N, n = 1, R³ 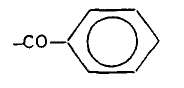 bedeutet;
= O, X = N, n = 2, R² und R³ 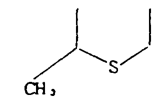 ist; und, wenn
Y = O, X = O, n = 1,
R⁸ und R⁹ je -CH₂-CH₂-Cl, R¹⁰ H₂ und R¹¹ + R¹² -CH₂-CH₂- oder -CH₂-CH₂-CH₂- bedeuten, wenn Y = S, X = N, R³ -CH₂-CH₂- ist, sowie deren Salzen mit pharmazeutisch verträglichen Säuren, zur Herstellung eines Arzneimittels zur Behandlung von Endokrinopathien.

2. Verwendung nach Anspruch 1, dadurch gekennzeichnet, daß als Phosphorderivate von Alkaloiden die Alkaloidderivate von Thiophosphorsäure der allgemeinen Formel 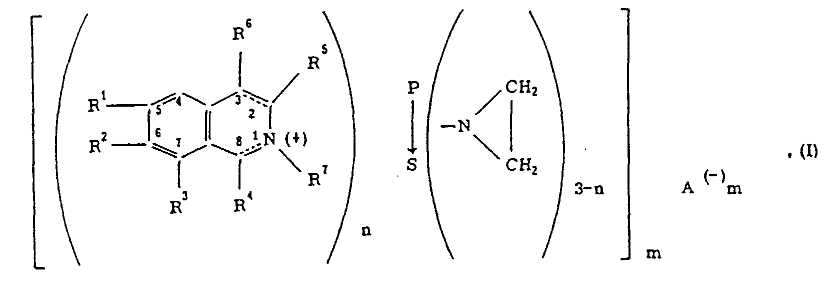 worin n 1, 2 oder 3 ist; m 1, 2 oder 3 bedeutet; R¹, R² und R³ unabhängig voneinander jeweils Wasserstoff oder Methoxy bedeuten, wobei R¹ und R² oder R² und R³ zusammen auch eine Methylendioxygruppe darstellen können; R⁴ Wasserstoff, Hydroxy oder Methyl bedeutet; und, wenn R⁶ Wasserstoff ist, R⁵ und R⁷ zusammen die Gruppe 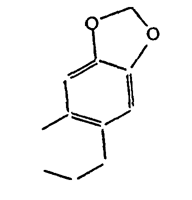 bilden; oder, wenn R⁷ eine Methylgruppe bedeutet, die Gruppen R⁵ und R⁶ die Gruppe 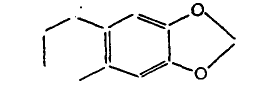 darstellen; und in Stellung 1, 8 und/oder 2, 3 eine Doppelbindung vorhanden sein kann; und A ein einwertiges oder den äquivalenten Teil eines mehrwertigen Anions bedeutet, eingesetzt werden.

3. Verwendung nach Anspruch 1 oder 2, dadurch gekennzeichnet, daß das Umsetzungsprodukt der Alkaloide von Chelidonium maijus L. mit Thiophosphorsäuretriaziridid eingesetzt wird.

4. Verwendung nach einem der Ansprüche 1 bis 3, dadurch gekennzeichnet, daß ein Arzneimittel zur Behandlung von Osteoporose hergestellt wird.

## Claims

1. The use of phosphorus derivatives of alkaloids of the general formula (I) 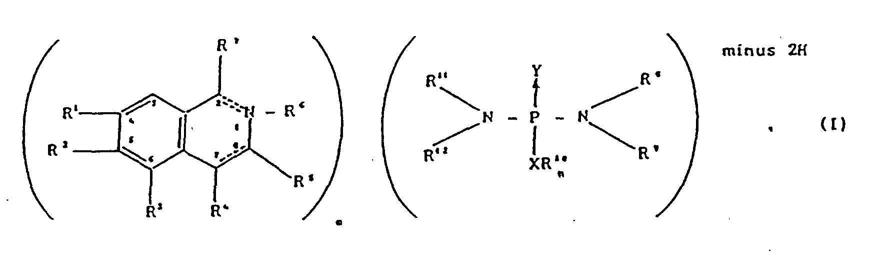 wherein m and n = 1, 2 or 3; R¹ R² and R³ are each independently H or methoxy, wherein R¹ and R², or R² and R³ together also may represent a methylene dioxy group;
R⁴ and R⁵, together with the C atoms, to which they are attached, form an optionally totally or partially hydrogenated phenyl or naphthyl group, which in turn may be substituted by methoxy, hydroxy or dioxymethyl, wherein R⁷ is H or =O or an equal ring system bonded via a -CH₂-CO-CH₂ chain, R⁶ is -CH₃, and double bonds may be present in positions 1, 2 and/or 7, 8; or
R⁶ and R⁷ together with the C and N atoms, to which they are attached, form an optionally hydrogenated benzo or naphtho ring system, which in turn may be substituted by methoxy, oxo, methyl or dioxymethyl groups, wherein the C-N bond in positions 1, 2 may be missing, and R⁴ and R⁵ are H;
R¹⁰ = 2H, -CH₂-CH₂-, H or -CH₂-CH₂Cl;
X = O or N,
R⁸ + R⁹ and R¹¹ + R¹² are -CH₂-CH₂- and, if Y = S, X = N and n = 2, R² and R³ are -CH₂-CH₂-, -CH₂-CH₂-O-CH₂-CH₂- or 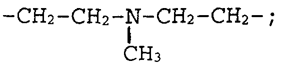 if
Y = S, X = N, n = 2, R² and R³ represent -CH₂-CH₂- , 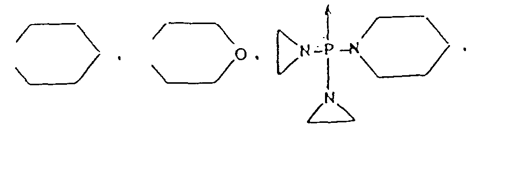 or (-C₂H₅)₂, 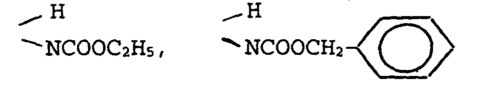 or 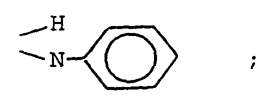 if
Y = S, X = O, n = 1, R³ is 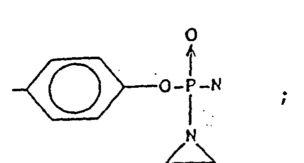 if
Y = O, X = N, n = 1, R³ is 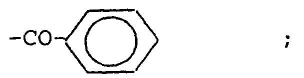 if
Y = O, X = N, n = 2, R² and R³ are 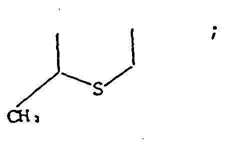 and if
Y = O, X = O, n = 1,
R⁸ and R⁹ are each -CH₂-CH₂-Cl, R¹⁰ is H₂ and R¹¹ + R¹² are -CH₂-CH₂- or -CH₂-CH₂-CH₂-, if Y = S, X = N, R³ is -CH₂-CH₂-, as well as their salts with pharmaceutically compatible acids, for the production of a medicament for the treatment of endocrinopathies.

2. The use according to claim 1, characterised in that as the phosphorus derivatives of alkaloids, the alkaloid derivatives of thiophosphoric acid of the general formula 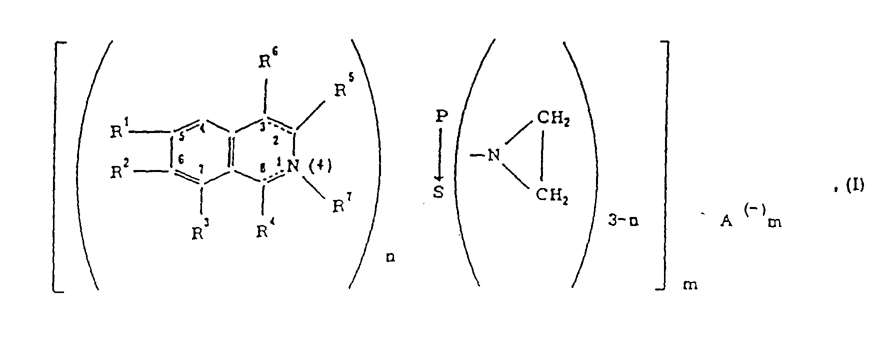 are used, wherein n is 1, 2 or 3; m represents 1, 2 or 3; R¹, R² and R³ each independently are hydrogen or methoxy, wherein R¹ and R² or R² and R³ together also may represent a methylene dioxy group; R⁴ is hydrogen, hydroxy or methyl; and, if R⁶ is hydrogen, R⁵ and R⁷ together form the group 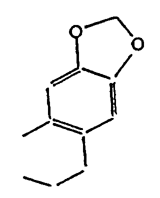 or, if R⁷ represents a methyl group, the groups R⁵ and R⁶ represent the group 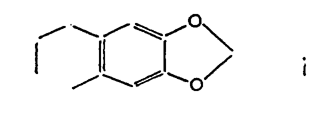 and in positions 1, 8 and/or 2, 3 there may be a double bond; and A is a monovalent or the equivalent portion of a polyvalent anion.

3. The use according to claim 1 or 2, characterised in that the reaction product of the alkaloids of Chelidonium maius L. with thiophosphoric acid triaziridide is used.

4. The use according to any one of claims 1 to 3, characterised in that a medicament for the treatment of osteoporosis is produced.

## Revendications

1. Utilisation de dérivés phosphorés d'alcaloïdes de formule générale (I) 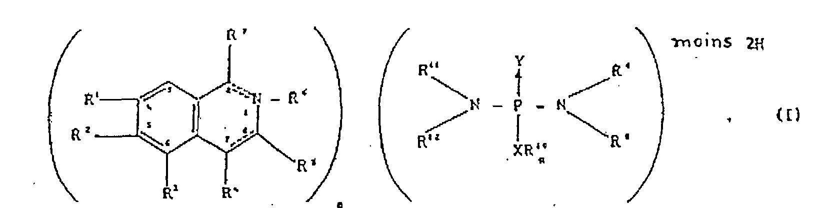 dans laquelle m et n sont égaux à 1, 2 ou 3 ; R¹, R² et R³ représentent, indépendamment les uns des autres, chacun H ou un groupe méthoxy, R¹ et R² ou bien R² ou R³ pouvant aussi former ensemble un groupe méthylène-dioxy ;
R⁴ et R⁵ forment, conjointement avec les atomes de carbone auxquels ils sont liés, un groupe phényle ou naphtyle le cas échéant entièrement ou partiellement déshydrogéné, qui peut être substitué de son côté par un reste méthoxy, hydroxy ou dioxyméthyle, R⁷ représente H ou =O ou un même système cyclique lié par l'intermédiaire d'une chaîne -CH₂-CO-CH₂-, R⁶ est un groupe -CH₃ et des doubles liaisons peuvent être présentes en positions 1, 2 et/ou 7, 8 ; ou bien
R⁶ et R⁷ forment, conjointement avec l'atome de carbone et l'atome d'azote auxquels ils sont liés, un cycle benzénique ou naphtalénique éventuellement hydrogéné, qui peut être substitué de son côté par des groupes méthoxy, oxo, méthyle ou dioxyméthyle, la liaison C-N en positions 1, 2 pouvant être manquante et R⁴ et R⁵ représentant H ;
R¹⁰ = 2H, -CH₂-CH₂-, H ou -CH₂-CH₂-Cl ;
X = O ou N ;
R⁸ + R⁹ et R¹¹ + R¹² représentent -CH₂-CH₂- et, lorsque Y = S, X = N et n = 2, R² et R³ représentent un groupe -CH₂-CH₂-, -CH₂-CH₂-O-CH₂-CH₂- ou 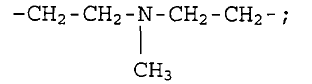
lorsque Y = S, X = N, n = 2, R² et R³ représentent -CH₂-CH₃-, 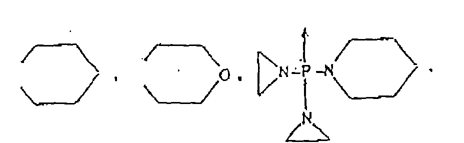
ou (-C₂H₅)₂, 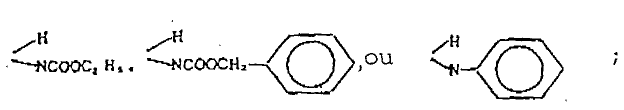 lorsque
Y = S, X = O, n = 1, R³ représente 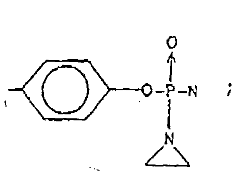 lorsque
Y = O, X = N, n = 1, R³ représente 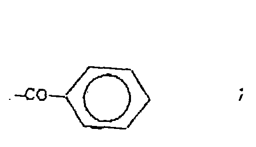 lorsque
Y = O, X = N, n = 2, R² et R³ représentent 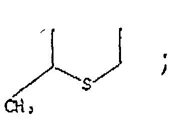 et, lorsque
Y = O, X = O, n = 1,
R⁸ et R⁹ représentent chacun un groupe -CH₂-CH₂-Cl, R¹⁰ représente H₂ et R¹¹ + R¹² représentent -CH₂-CH₂- ou -CH₂-CH₂-CH₂-, lorsque Y = S, X = N, R³ représente un groupe -CH₂-CH₂-, ainsi que leurs sels d'acides acceptables du point de vue pharmaceutique, pour la préparation d'un médicament destiné au traitement d'endocrinopathies.

2. Utilisation suivant la revendication 1, caractérisée en ce qu'on utilise comme dérivés phosphorés d'alcaloïdes les dérivés d'alcaloïdes de l'acide thiophosphorique de formule générale 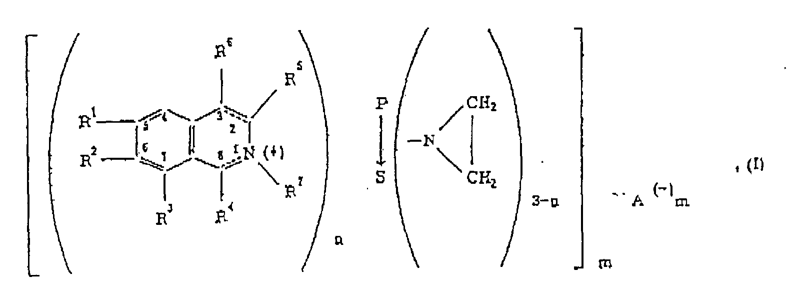 dans laquelle n est égal à 1, 2 ou 3 ; m est égal à 1, 2 ou 3 ; R¹, R² et R³ représentent chacun, indépendamment les uns des autres, l'hydrogène ou un groupe méthoxy, R¹ et R² ou bien R² et R³ pouvant aussi former ensemble un groupe méthylène-dioxy ; R⁴ est l'hydrogène, un groupe hydroxy ou méthyle ; et lorsque R⁶ est l'hydrogène, R⁵ et R⁷ forment ensemble le groupe 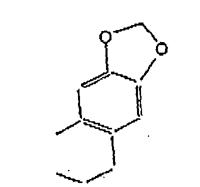 ou bien lorsque R⁷ est un groupe méthyle, les groupes R⁵ et R⁶ forment le groupe 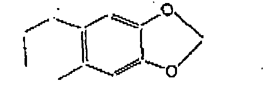 et une double liaison peut être présente en positions 1,8 et/ou 2,3 ; et A désigne un anion monovalent ou la partie équivalente d'un anion polyvalent.

3. Utilisation suivant la revendication 1 ou 2, caractérisée en ce qu'on utilise le produit de réaction des alcaloïdes de Chelidonium maijus L. avec le triaziridide de l'acide thiophosphorique.

4. Utilisation suivant l'une quelconque des revendications 1 à 3, caractérisée en ce qu'on prépare un médicament destiné au traitement de l'ostéoporose.
